# FASCICULE DE BREVET EUROPEEN

(11) **EP 0 625 036 B1**
(45) Date de publication et mention de la délivrance du brevet: **13.11.1996**
(21) Numéro de dépôt: 93904148.9
(22) Date de dépôt: 02.02.1993
(51) Int. Cl.: A61F 15/00

(54) **PROTECTION DE BANDAGE OU ANALOGUES**
SCHUTZHUELLE FUER VERBAENDEN ODER DERGLEICHEN
PROTECTION OF BANDAGES OR SIMILAR

(30) Priorité: 03.02.1992 FR 9201174
(43) Date de publication de la demande: 23.11.1994
(73) Titulaire: FAURE, Camille, F-21800 Quetigny (FR)
(72) Inventeur: FAURE, Camille, F-21800 Quetigny (FR)
(74) Mandataire: Bruder, Michel
(86) Numéro de dépôt international: FR9300107
(87) Numéro de publication internationale: WO9314730

(56) Documents cités:
- US-A- 4 178 924
- US-A- 4 423 722
- US-A- 4 562 834
- US-A- 4 966 135

## Description

La présente invention concerne un équipement de protection sanitaire notamment destiné à préserver les bandages et pansements protégeant par exemple, le siège d'une lésion d'un membre, permettant notamment l'usage normal de la douche pour les personnes atteintes d'une telle lésion.

A cet égard, on connaît déjà de nombreux équipements assurant une telle protection, c'est par exemple, le cas d'un doigtier ou d'une genouillère qui ont pour principal intérêt d'assurer une protection contre les effets mécaniques c'est le cas du bonnet de douche qui assure une protection de la chevelure contre les projections verticales d'eau ; il existe également de nombreux gants de protection, qu'il s'agisse de gants de ménage ou de gants de chirurgien dont le but est toujours d'isoler le membre de l'environnement extérieur réputé agressif.

Tous ces dispositifs protecteurs sont en général conçus pour convenir au plus grand nombre selon une taille unique dans un souci évident d'universalité. Naturellement, il résulte de ces dispositifs, soit une évidente inadaptation, soit un inconfort et, on trouve à ce titre dans la littérature, quelques propositions d'équipement adaptables dans le domaine de la protection des bandages et pansements. Dans ce sens, le brevet américain US-4.562.834 propose un gant et un dispositif de protection particulièrement destiné aux mains et aux pieds dont une caractéristique principale est de pouvoir être ajusté en longueur et même en diamètre par enlevage de tranches de matières successives pré-découpées à l'origine ; pour l'essentiel, ces dispositifs de protection ajustables sont particulièrement avantageux lorsqu'il s'agit d'adapter ledit dispositif en longueur ou en profondeur. En outre, ces mêmes dispositifs ainsi d'ailleurs que les premiers cités, n'assurent que très accessoirement une étanchéité suffisante pour offrir aux utilisateurs souffrant de plaies en cours de traitement, de faire normalement usage, par exemple, de la douche pour des soins quotidiens d'hygiène corporelle ; on comprend bien à cet égard, qu'il soit fortement proscrit de souiller et, en tout cas, de mouiller les pansements qui protègent une plaie à l'occasion de telles ablutions.

Le brevet américain US-4.423.722 propose quant à lui un équipement de protection de bandages comportant une entrée incluant une partie tubulaire fermée d'un côté et, ouverte dans sa partie supérieure suivant un cône pouvant s'ajuster sur le membre portant le bandage. L'ensemble étant naturellement en une matière élastique venant enserrer le membre. Ce dispositif présente néanmoins l'inconvénient de n'être pas ajustable, d'abord en longueur et, surtout, dans sa partie de serrage annulaire dont le seul réglage résulte de l'élasticité du matériau de fabrication.

La présente invention vise donc à pallier tous les inconvénients qui viennent d'être cités en proposant un équipement de protection contre les projections, par exemple, d'eau ou contre les souillures d'une lésion d'un membre ou des bandages protégeant ladite lésion. L'équipement est réalisé en un matériau élastique et étanche, selon une forme sensiblement tubulaire dont l'une au moins de ses extrémités est ouverte, et de forme approximativement tronconique dont la section d'entrée, correspondant au sommet du tronc de cône, est obtenue par une opération de coupe radiale, à une hauteur prédéterminée dudit tronc, convenable pour procurer un serrage périphérique du membre comportant la lésion, suffisant pour une étanchéité à l'eau.

A tel fin, dans l'équipement selon l'invention, il est prévu d'adjoindre sur l'extrémité tronconique du dispositif, des indications dimensionnelles permettant de repérer avec précision la zone de coupe souhaitée, précisant finalement la section de l'orifice d'entrée de l'équipement de protection.

Une variante selon l'invention concerne l'utilisation d'équipement de protection, en cas de lésion du pied ou pour la protection de bandages et autres pansements analogues situés dans le bas des jambes ; dans cette variante il est prévu d'adjoindre à l'extrémité d'entrée de l'équipement de protection une obturation pouvant, le cas échéant, avoir la forme générale d'un pied de telle manière que l'ensemble de l'équipement disposé en bout de la jambe soit totalement étanche ; il est, à cet égard, prévu que la partie inférieure constituant l'obturation de l'équipement destiné à la protection des lésions sur le pied comporte extérieurement un relief anti-dérapant assurant ainsi la sécurité, notamment dans les bacs à douche.

Une autre variante de l'équipement de protection suivant l'invention consiste à aménager, de préférence, dans la partie centrale de l'équipement, un soufflet permettant d'adapter dans une certaine mesure la forme et la longueur de l'équipement en fonction de la morphologie du membre et, de la taille éventuelle des lésions ou bandages à protéger.

D'autres caractéristiques et avantages ressortiront mieux de la description qui va suivre qui n'est donnée qu'à titre d'exemple non limitatif de mise en oeuvre de l'invention en référence aux dessins annexés sur lesquels :
- La figure 1 représente une vue frontale de l'équipement selon l'invention, dans sa variante la plus simple.
- La figure 2 est une vue frontale d'une variante de l'équipement de protection muni de son soufflet d'extension longitudinal et montrant les repères dimensionnels dans la partie tronconique d'entrée.
- La figure 3 représente une vue schématique de l'équipement disposée selon une variante obturée à l'extrémité et aménagée pour la protection des lésions au pied.

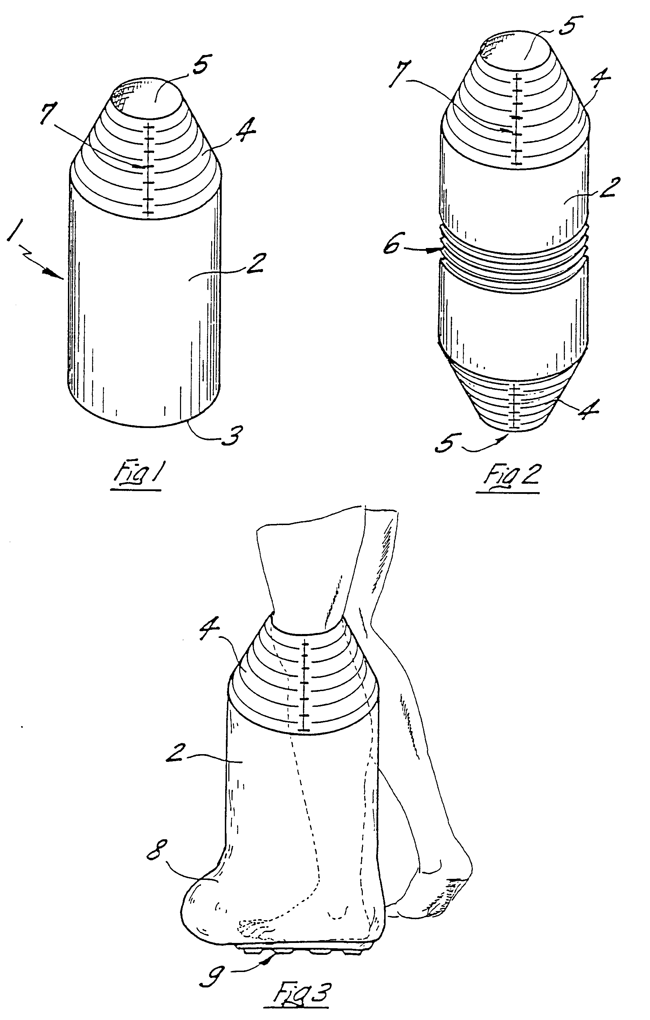

Le dispositif 1 conforme à l'invention, représenté dans sa version élémentaire sur la figure 1 est constitué d'une partie tubulaire 2 réalisée en un matériau élastique, de préférence en latex ou tout autre matériau tel que le caoutchouc synthétique, dont l'épaisseur est Variable comme il sera dit plus loin. La partie tubulaire 2 du dispositif 1 est ouverte en son extrémité inférieure 3 pour permettre le passage du membre dont on veut protéger la lésion. L'extrémité supérieure 4 dudit corps 2 est de forme générale tronconique, la partie extrême 5 étant ouverte pour constituer la zone d'entrée du membre dont la lésion est à protéger. On comprend bien dès lors, qu'une coupe radiale effectuée à hauteur convenable du tronc de cône 4, procure à l'équipement une zone d'entrée 5 de dimension variable ; on peut ainsi adapter ladite section d'entrée de l'équipement de protection à la morphologie et, en particulier, au périmètre du membre que l'on veut protéger. En réalité on s'arrangera pour obtenir un périmètre d'entrée légèrement inférieur au périmètre mesuré du membre que l'on entend protéger, de telle manière qu'il existe finalement un serrage suffisant pour maintenir en place l'équipement de protection. On notera que des essais concluants ont été effectués avec un équipement dont le diamètre de la partie tubulaire se situait aux environs de 20 cm et dont la section de la zone d'entrée 5 était prévue avant découpe aux environs de 5 cm.

Dans une variante conformément à la figure 2, il est prévu de disposer dans la partie tubulaire de l'équipement 1, un soufflet 6 permettant d'obtenir une variation longitudinale de la partie tubulaire 2 de l'équipement 1. Le soufflet 6 est réalisé d'une manière tout à fait connue en soi, il peut être rapporté par soudure ou obtenu directement par moulage suivant des techniques à la portée de l'homme du métier. Le soufflet 6 peut en variante être totalement élastique ou à soufflets dépliables et repliables suivant un certain nombre de plis initialement prévus afin d'obtenir une variation de courbure et/ou de longueur dépendant des zones de protection envisagées.

Il est bien évident que en variante particulière, l'extrémité inférieure 3 du dispositif 1 peut être réalisée de la même manière que l'extrémité 4 c'est à dire en forme de tronc de cône dont il serait alors possible, par des coupes radiales, de régler comme pour l'entrée, la section de sortie créant ainsi une zone intermédiaire pour le membre ou la lésion à protéger ; naturellement dans cette dernière variante on comprend mieux encore les avantages d'un ajustement en longueur de la partie tubulaire grâce au soufflet 6.

Sur l'équipement suivant l'invention précédemment décrites, il est prévu d'inscrire par exemple par un procédé de sérigraphie ou directement par moulage au moment de la réalisation de l'équipement, des repères dimensionnels 7 permettant d'indiquer clairement les zones de coupe convenables pour la morphologie du membre à protéger ; il est par exemple prévu de faire figurer, sur une génératrice du tronc de cône, un filet délimitant une section du tronc de cône et comportant en cm la dimension ainsi obtenue de la zone d'entrée correspondante et, éventuellement de la zone de sortie.

Dans une dernière variante de réalisation de l'équipement selon l'invention et, en référence à la figure 3, l'extrémité inférieure, ouverte dans la variante principale, est ici remplacée par une excroissance fermée 8 en forme générale permettant d'accueillir de manière confortable, un pied. Il est prévu de rajouter avantageusement sur la partie de l'excroissance 8 devant être en contact avec le sol, une surface anti-dérapante 9 celle-ci peut être obtenue par moulage direct d'un simple relief.

Il est enfin prévu de réaliser une version jetable des équipements de protection selon l'invention et, à cet égard, il a été retenu une épaisseur du matériau élastique étanche de l'ordre du dixième de millimètre en version jetable et de l'ordre de quelques dixièmes pour la version réutilisable.

## Revendications

1. Equipement de protection contre les projections notamment d'eau ou contre les souillures d'une lésion d'un membre ou des bandages protégeant ladite lésion ou similaire, réalisé en un matériau élastique et étanche selon une forme sensiblement tubulaire dont l'une au moins des extrémités est ouverte et de forme générale tronconique caractérisé en ce que ladite extrémité tronconique porte sur sa surface extérieure des indications dimensionnelles (7) permettantes de repérer avec précision, lors de l'adaptation moyennant une decoupe radiale de la section d'entrée et/ou de sortie de l'équipement correspondant au sommet (5) du tronc de cône (4), la zone de coupe sur ledit tronc (4) a un serrage périphérique du membre comportant la lésion suffisant pour une efficace étancheité à l'eau.

2. Equipement de protection selon la revendication 1 caractérisé en ce que les indications dimensionnelles (7) sont imprimées ou directement moulées dans la matière.

3. Equipement de protection selon l'une quelconque des revendications précédentes caractérisé en ce que l'extrémité (3) opposée à la partie tronconique d'entrée (4) est obturée de manière étanche.

4. Equipement de protection selon la revendication précédente caractérisé en ce que son extrémité terminale présente une excroissance (8) en forme générale de pied.

5. Equipement de protection selon la revendication précédente en ce que la partie inférieure de l'excroissance (8) en contact avec le sol, comporte un relief anti-dérapant (9).

6. Equipement de protection selon l'une quelconque des revendications précédentes caractérisé en ce qu'un soufflet (6) est prévu dans la partie tubulaire (2) dudit équipement (1) permettant un allongement reversible, élastique ou non, suivant l'axe longitudinal dudit équipement (1).

## Claims

1. Equipment providing protection against splashes of water in particular or against dirt for a wound in a limb or bandages protecting the said wound or the like, constructed of elastic watertight material of a substantially tubular shape, at least one end of which is open and has a generally frustoconical shape characterised in that the said frustoconical end bears dimensional information (7) on its outer surface providing accurate reference marks when it is fitted, using a radial cut in the equipment's entrance and/or exit section corresponding to the top (5) of the frustum of the cone (4), the cut area in the said frustum (4) forming a peripheral seal against the limb bearing the wound which is sufficient to provide effective watertightness.

2. Protective equipment according to claim 1, characterised in that the dimensional reference marks (7) are printed or directly moulded within the material.

3. Protective equipment according to any one of the foregoing claims, characterised in that the end (3) opposite the entry frustoconical part (4) is closed off so as to form a seal.

4. Protective equipment according to the foregoing claim, characterised in that its terminal end has an excrescence (8) in the general form of a foot.

5. Protective equipment according to the foregoing claim in which the lower part of the excrescence (8) in contact with the ground incorporates a non-slip thread (9).

6. Protective equipment according to any one of the foregoing claims characterised in that the corrugated part (6) is provided in the tubular part (2) of the said equipment (1) to permit elastic or non-elastic reversible elongation along the longitudinal axis of the said equipment (1).

## Patentansprüche

1. Schutzeinrichtung gegen Spritzer, insbesondere Wasserspritzer, oder gegen Verunreinigungen einer Verletzung eines Gliedes oder von Binden, welche die besagte Verletzung schützen, oder dergleichen, die aus einem elastischen und dichten Material gemaß einer im wesentlichen röhrenförmigen Form gefertigt ist, von deren Enden mindestens das eine offen und von allgemein kegelstumpfförmiger Gestalt ist, dadurch gekennzeichnet, daß das besagte kegelstumpfförmige Ende auf seiner Außenseite Maßangaben (7) trägt, die es gestatten, bei der Anpassung mittels eines radialen Schnitts mit Genauigkeit den Eintritts- und/oder Austrittsquerschnitt der Einrichtung Zu bestimmen, welcher der Spitze (5) des Kegelstumpfs (4) entspricht, wobei die Schnittzone auf dem besagten Stumpf (4) eine Umfangsanpressung gegen das die Verletzung aufweisende Glied besitzt, die für eine wirksame Wasserdichtheit ausreicht.

2. Schutzeinrichtung nach Anspruch 1, dadurch gekennzeichnet, daß die Maßangaben (7) aufgedruckt oder unmittelbar in das Material eingeformt sind.

3. Schutzeinrichtung nach einem beliebigen der vorangehenden Ansprüche, dadurch gekennzeichnet, daß das zum kegelstumpfförmigen Eintrittsteil (4) entgegengesetzte Ende (3) dicht verschlossen ist.

4. Schutzeinrichtung nach dem vorangehenden Anspruch, dadurch gekennzeichnet, daß sein äußerstes Ende einen Auswuchs (8) in einer allgemeinen Fußform aufweist.

5. Schutzeinrichtung nach dem Vorangehenden Anspruch, dadurch gekennzeichnet, daß der mit dem Boden in Berührung befindliche untere Teil des Auswuchses (8) ein Anti-Rutschprofil (9) aufweist.

6. Schutzeinrichtung nach einem beliebigen der vorangehenden Ansprüche, dadurch gekennzeichnet, daß im röhrenförmigen Teil (2) der besagten Einrichtung (1) ein Balg (6) vorgesehen ist, der eine elastische oder nichtelastische reversible Verlängerung entlang der Längsachse der besagten Einrichtung (1) gestattet.
